# EUROPÄISCHE PATENTSCHRIFT

(11) **EP 2 782 503 B1**
(45) Veröffentlichungstag und Bekanntmachung des Hinweises auf die Patenterteilung: **12.04.2017**
(21) Anmeldenummer: 12797787.4
(22) Anmeldetag: 16.11.2012
(51) Int. Cl.: A61B 5/18, B62D 1/04, A61B 5/0295, A61B 5/0402, A61B 5/1455, A61B 5/024, A61B 5/0404

(54) **ERFASSUNG VON VITALPARAMETERN MIT EINEM OPTISCHEN SENSOR AM LENKRAD**
DETECTION OF VITAL PARAMETERS BY MEANS OF AN OPTICAL SENSOR ON THE STEERING WHEEL
DÉTECTION DE PARAMÈTRES VITAUX AU MOYEN D'UN CAPTEUR OPTIQUE MONTÉ SUR LE VOLANT

(30) Priorität: 21.11.2011 DE 102011086740
(43) Veröffentlichungstag der Anmeldung: 01.10.2014
(73) Patentinhaber: ZF Friedrichshafen AG, 88046 Friedrichshafen (DE)
(72) Erfinder: RAKE, Ludger, 49439 Steinfeld (DE); GIEFER, Andreas, 49448 Lemfoerde (DE); MEYER, Joerg, 49419 Wagenfeld (DE)
(86) Internationale Anmeldenummer: PCT/EP2012/072867
(87) Internationale Veröffentlichungsnummer: WO 2013/076018

(56) Entgegenhaltungen:
- DE-A1-102005 007 963
- US-A1- 2006 025 698
- US-A1- 2008 238 695
- US-A1- 2011 245 643
- FUTATSUYAMA ET AL: "Noise Robust Optical Sensor for Driver's Vital Signs", SP-2299, SAE 2011 WORLD CONGRESS & EXHIBITION, 12. April 2011 (2011-04-12), Seiten 1-9, XP009167777,
- HANBIT PARK ET AL: "Drowsy driving detection based on human pulse wave by photoplethysmography signal processing", PROCEEDINGS OF THE 3RD INTERNATIONAL UNIVERSAL COMMUNICATION SYMPOSIUM (IUCS '09), 1. Januar 2009 (2009-01-01), Seiten 89-92, XP055055678, New York, New York, USA DOI: 10.1145/1667780.1667798 ISBN: 978-1-60-558641-0
- STEPHAN HEUER ET AL: "Unobtrusive in-vehicle biosignal instrumentation for advanced driver assistance and active safety", 2010 IEEE EMBS CONFERENCE ON BIOMEDICAL ENGINEERING AND SCIENCES (IECBES 2010), 30. November 2010 (2010-11-30), Seiten 252-256, XP031936758, DOI: 10.1109/IECBES.2010.5742238 ISBN: 978-1-4244-7599-5

## Beschreibung

Die Erfindung betrifft eine Erfassungsvorrichtung zur Erfassung zumindest eines Vitalparameters einer Person in einem Kraftfahrzeug, gemäß dem Oberbegriff des Patentanspruchs 1.

Eine solche Erfassungsvorrichtung ist beispielsweise aus den Dokumenten DE 10 2005 007 963 A1, US 2006/025698 A1 und US 2008/238695 A1 bekannt.

Die Erfassung von Vitalparametern von Personen mittels optischer Plethysmographie (Messung von Volumenschwankungen) und Pulsoxymetrie (Messung der Sauerstoffsättigung) ist für sich genommen bekannt, und erfolgt üblicherweise mittels nicht-invasiver Ermittlung insbesondere der Pulsrate, der Pulsratenvariabilität und der arteriellen Sauerstoffsättigung anhand der Messung der Lichtabsorption bzw. Lichtreflektion beim Lichtdurchgang durch das Gewebe.

Plethysmographie und Pulsoxymetrie basieren dabei grundsätzlich auf ähnlichen optischen Messverfahren. Die Plethysmographie erfolgt anhand einer optischen Messung der Änderung der Lichtabsorption, die sich durch Volumenschwankungen insbesondere von Blutgefäßen ergibt, während die Pulsoxymetrie auf der - in Abhängigkeit der Blutsauerstoffsättigung - unterschiedlichen Lichtabsorption bzw. Lichtremission eines roten sowie eines infraroten Messlichtstrahls bei Durchleuchtung der Haut und des Gewebes basiert.

Typischerweise werden bei der Pulsoxymetrie die Sauerstoffsättigungswerte (SPO2-Werte) über einen optischen Sensor am Finger, Zeh oder Ohrläppchen abgenommen, wobei die Messung zumeist mit einem Clipsensor oder Klebesensor erfolgt. Die dabei verwendeten Sensoren bestehen üblicherweise aus einer oder zwei Lichtquellen, z.B. in Form einer roten Diode und/oder einer infraroten Diode, im Zusammenhang mit einem Fotosensor bzw. einer Fotodiode. Die infrarote Diode strahlt dabei im nicht-sichtbaren Bereich des elektromagnetischen Spektrums, während die rote Diode Licht im sichtbaren Bereich emittiert.

Durch die unterschiedliche Färbung des mit Sauerstoff mehr oder weniger gesättigten Blut-Hämoglobins entstehen für das durchstrahlende Rotlicht bzw. Infrarotlicht unterschiedliche Absorptionsgrade, die durch den Fotosensor aufgenommen werden. Dabei kann eine Auswerteeinheit - beispielsweise mittels Vergleich der Messergebnisse mit einer Referenztabelle - die Sauerstoffsättigung des Bluts in den Kapillaren erfassen bzw. bestimmen. Neben der Sauerstoffsättigung kann mittels der optoelektronischen Sensoranordnung generell auch der Puls bzw. die Pulswelle, Pulsrate und die Pulsratenvariabilität bestimmt werden.

Dabei sind optische Plethysmographie und Pulsoxymetrie Bestandteil des klinischen Alltags und werden sowohl für die Standardüberwachung von Patienten als auch für diagnostische Zwecke verwendet. Unter anderem wird die Pulsoxymetrie jedoch auch immer mehr für "Homecare", also für die gesundheitliche Überwachung des Patienten z.B. in der häuslichen Umgebung eingesetzt. Dies betrifft insbesondere die Betreuung von Patienten mit kardiologischen Risikofaktoren sowie die Diagnostik von Schlafstörungen und die Erkennung von Müdigkeits- bzw. Stresszuständen.

Die Anwendung der optischen Plethysmographie (Messung von Volumenschwankungen) und Pulsoxymetrie (Messung der Sauerstoffsättigung) zum Zweck der Erfassung der genannten Vitalparameter von Patienten bzw. Personen kann jedoch nicht nur unter stationären Bedingungen oder im Krankenhaus, sondern auch unterwegs, also beispielsweise während der Fahrt in einem Automobil bzw. Kraftfahrzeug durchgeführt werden.

Für solche nicht-stationäre Messungen sind prinzipiell mobile Geräte bekannt, deren Sensorik jeweils direkt am Körper angebracht wird. Hierdurch wird jedoch die Bewegungsfreiheit der Patienten eingeschränkt, weshalb derartige Anwendungen bzw. Geräte als Lösung insbesondere in einem Automobil häufig nicht akzeptabel sind. Um der Problematik der eingeschränkten Bewegungsfreiheit unter Automobilbedingungen zu begegnen, ist daher bereits versucht worden, die genannten optischen Sensoren in Bedienelemente des Kraftfahrzeugs zu integrieren. Ziel ist dabei stets eine Erfassung der Vitalparameter mit möglichst geringer Beeinträchtigung des Fahrers.

Aus der DE 10 2008 056 250 A1 ist es bekannt, optische Sensoren für die Plethysmographie bzw. Pulsoxymetrie in einen Schaltknauf eines Kraftfahrzeugs zu integrieren. Bei diesem aus dem Stand der Technik bekannten Lösung muss der Fahrer zur Durchführung der Messung zunächst eine Hand vom Lenkrad nehmen, weshalb die Messung u.U. nicht so häufig bzw. regelmäßig durchgeführt werden kann, wie dies im Hinblick auf das Monitoring des Gesundheitszustands des Fahrers wünschenswert ist. Aus diesem Grund wurde bereits vorgeschlagen, optische Sensoren für die zuvor genannten Messverfahren in den Lenkradkranz eines Kraftfahrzeugs zu integrieren. Bei einer Anordnung im Lenkradkranz tritt jedoch häufig eine unzureichende Signalqualität der optischen Messung auf, insbesondere aufgrund der dort knappen Platzverhältnisse, der starken möglichen Helligkeitsschwankungen aufgrund der Nähe zur Windschutzscheibe, sowie aufgrund der schwierig reproduzierbaren, für die Messung erforderlichen Fingerhaltung des Fahrers.

Aus dem Stand der Technik ist es ferner bekannt, in einem Automobil während der Fahrt - alternativ oder zusätzlich zu plethysmographischen bzw. pulsoxymetrischen Messungen - auch EKG-Messungen durchzuführen. Dabei ist bereits versucht worden, auch die hierzu notwendigen EKG-Elektroden am Lenkrad, insbesondere im Bereich der Lenkrad-Oberseite am Lenkradkranz anzuordnen. Mit dieser Anordnung sind jedoch ebenfalls Nachteile im Hinblick auf die Gestaltung des Lenkrads ebenso wie Fluktuationen der Messwerte beim starken Aufheizen des Lenkrads bzw. der EKG-Sensoren durch Sonneneinstrahlung verbunden.

Mit der vorliegenden Erfindung soll eine Vorrichtung zur Erfassung von Vitalparametern geschaffen werden, mit der die genannten Nachteile bzw. Einschränkungen überwunden werden sollen. Mit der Erfindung soll dabei insbesondere eine zuverlässige und im Betrieb robuste Integration der Messsensoren zur Ermittlung des Blutsauerstoffgehalts, der Pulscharakteristik und/oder des Elektrokardiogramms des Fahrers eines Kraftfahrzeugs ermöglicht werden. Insbesondere sollen die beschriebenen Nachteile der Anordnung der Messsensoren im Bereich des Schaltknaufs eines Kraftfahrzeugs und/oder auf dem Lenkradkranz mit der Erfindung überwunden werden.

Dazu schlägt die vorliegende Erfindung eine Erfassungsvorrichtung mit den Merkmalen des Patentanspruchs 1 vor.

Bevorzugte Ausführungsformen sind Gegenstand der Unteransprüche.

Die Erfassungsvorrichtung gemäß der vorliegenden Erfindung dient zur Erfassung zumindest eines Vitalparameters einer Person in einem Kraftfahrzeug. Hierzu weist die Erfassungsvorrichtung zumindest eine Fingersensoreinrichtung mit zumindest einer optischen Sensorvorrichtung und einen zugeordneten Tastschalter auf, wobei die Sensoreinrichtung zumindest eine erste Lichtquelle sowie zumindest ein lichtempfindliches Element umfasst.

Die Fingersensoreinrichtung ist am Lenkrad im Übergangsbereich zwischen einer Lenkradspeiche und dem Lenkradkörper bzw. Lenkrad-Pralltopf angeordnet. Üblicherweise ist ein Lenkrad derart aufgebaut, dass der im Zentrum des Lenkrades angeordnete Lenkradkörper bzw. Lenkrad-Pralltopf bzw. Lenkradnabe zur Verbindung mit einer Lenksäule zur Übertragung der mit dem Lenkrad durchgeführten Lenkbewegung auf die Lenksäule vorgesehen ist, wobei der Lenkradkörper über Lenkradspeichen mit einem den Lenkradkörper dazu beabstandet umlaufenden, meist kreisförmigen Lenkradkranz verbunden ist. Der Lenkradkörper ist üblicherweise dazu vorgesehen, eine als Hupe ausgebildete Betätigungseinrichtung sowie je nach Kfz-Ausführung einen Fahrer-Airbag aufzunehmen. Der Lenkradkranz bildet ein Betätigungselement für den Fahrzeugführer bzw. Fahrer zum Aufnehmen und Übertragen der gewünschten Lenkbewegung aus. Die auf den Lenkradkranz ausgeübte Lenkbewegung wird über eine oder mehrere als starre Verbindung zwischen Lenkradkranz und Lenkradkörper ausgestaltete Lenkradspeichen auf den Lenkradkörper und über diesen auf die Lenksäule übertragen.

Bekanntermaßen weisen übliche Lenkräder darüber hinaus Bedienelemente in Form von Tastern oder Schaltern zum Fernbedienen einer Radioeinrichtung, einer Navigationseinrichtung, einer Klimaanlage, eines Bordcomputers, einer Geschwindigkeitsregeleinrichtung, einer Freisprecheinrichtung oder einer sonstigen Bedieneinrichtung auf. Mit anderen Worten wird es dem Fahrer über die am Lenkrad angeordneten Bedienelemente ermöglicht, die Bedienung der jeweiligen Bedieneinrichtung vorzunehmen, ohne seine Hand von dem Lenkrad nehmen zu müssen bzw. ohne eine Standardposition der Hände am Lenkrad verändern zu müssen. Dadurch kann eine ansonsten mit der unmittelbar an der Einrichtung vorgenommenen Bedienung der jeweiligen Bedieneinrichtungen einhergehende übliche Ablenkung des Fahrers minimiert werden.

Der Taster und/oder Schalter sind üblicherweise seitlich des Lenkradkörpers, mit anderen Worten an dem Lenkradkörper in einem Zwischenraum zwischen dem Lenkradkörper und dem Lenkradkranz angeordnet. Alternativ oder zusätzlich können solche Taster und Schalter in einem sich zwischen dem Lenkradkörper und der Lenkradspeiche erstreckenden Übergangsbereich angeordnet sein. Die Lenkradspeiche erstreckt sich dabei von dem Lenkradkranz in Richtung des Lenkradkörpers und ist mittels des Übergangsbereiches mit dem Lenkradkörper starr verbunden. Der Übergangsbereich kann bauteilseitig einen Teil der Lenkradspeiche und/oder einen Teil des Lenkradkörpers ausformen. Mit anderen Worten kann der Übergangsbereich einen Endbereich der Lenkradspeiche, an welchem Endbereich der Lenkradkörper angrenzt, oder einen Endbereich des Lenkradkörpers, an welchem Endbereich die Lenkradspeiche angrenzt, ausformen, wobei die Lenkradspeiche und der Lenkradkörper miteinander verbunden sind. Denkbar ist ferner, dass ein Teil des Übergangsbereiches durch die Lenkradspeiche und der andere Teil des Übergangsbereiches durch den Lenkradkörper ausgeformt sind, wobei die Lenkradspeiche und der Lenkradkörper in dem Übergangsbereich aneinander angrenzen und miteinander verbunden sind. Der Übergangsbereich ist dabei durch die Eigenschaft gekennzeichnet, dass in dem Übergangsbereich ein oder mehrere Bedienelemente zum Fernbedienen von Bedieneinrichtungen, wie beispielsweise den vorgenannten, angeordnet oder anordenbar sind, wobei die Bedienung des Bedienelementes oder der Bedienelemente von dem Fahrer ohne Loslassen des Lenkrades, weiter bevorzugt ohne Verändern einer Standardposition seiner Hände am Lenkrad vornehmbar ist.

Die Anordnung der Fingersensoreinrichtung am Lenkrad im Übergangsbereich zwischen einer Lenkradspeiche und dem Lenkradkörper hat den Vorteil, dass der Fahrer zur Messung der zu ermittelnden Vitalparameter, also beispielsweise zur Ermittlung des Blutsauerstoffgehalts oder des Pulses, die Hand nicht mehr vom Lenkrad nehmen und diese an dem beispielsweise im Bereich des Schalthebels angeordneten Messsensor anlegen muss. Vielmehr kann dank der Erfindung die Messung des bzw. der Vitalparameter mit geringstmöglichem Aufwand lediglich durch Anlage beispielsweise eines Fingers im Bereich der am Lenkrad angeordneten Fingersensoreinrichtung erfolgen. Infolge der Anordnung der Fingersensoreinrichtung im Übergangsbereich zwischen Lenkradspeiche und Lenkradkörper ergibt sich zudem der Vorteil, dass die Messung erfolgen kann, ohne die Hand vom Lenkradkranz nehmen zu müssen.

Die dadurch erleichterte Messung führt somit dazu, dass die Messungen einfacher und damit häufiger und regelmäßiger durchgeführt werden können, was insbesondere bei Patienten mit entsprechenden Risikofaktoren von Bedeutung sein kann.

Die Anordnung der Fingersensoreinrichtung am Lenkrad im Übergangsbereich zwischen einer Lenkradspeiche und dem Lenkradkörper bzw. Pralltopf hat den zusätzlichen Vorteil, dass dieser Abschnitt des Lenkrads üblicherweise nicht im unmittelbaren Bereich der Sonneneinstrahlung liegt, wodurch die beim Stand der Technik noch auftretende Fehllichteinstreuung in die Sensorik, bzw. die beim Stand der Technik ebenfalls beobachtete starke Aufheizung der am Lenkrad angeordneten Kontaktoberflächen verringert werden können.

Die Fingersensoreinrichtung kann dabei insbesondere so positioniert werden, dass die entsprechenden Sensorflächen - ohne zusätzliche Handbewegungen - bereits in der Standardhaltung des Lenkrads durch die Hände des Fahrers erreichbar sind. Dies kommt einer mit geringstmöglichem Aufwand durchführbaren Messung und damit der Effektivität der Vitalparameter-Erfassung zugute.

Gemäß bevorzugter Ausführungsformen kann die Fingersensoreinrichtung dabei auf der dem Fahrer abgewandten Rückseite des Lenkrads angeordnet und zur Kontaktierung zumindest eines Fingers (ausgenommen des Daumens) eingerichtet sein, oder die Fingersensoreinrichtung kann auf der dem Fahrer zugewandten Vorderseite des Lenkrads angeordnet und zur Kontaktierung eines Daumens eingerichtet sein. In beiden Fällen kann die Anlage des Fingers bzw. Daumens an der Fingersensoreinrichtung erfolgen, ohne dass die Hand vom Lenkrad genommen werden muss, und ohne dass der Lenkradkranz losgelassen werden muss.

Die Erfindung kann unabhängig davon verwirklicht werden, wie die Fingersensoreinrichtung konstruktiv im einzelnen ausgebildet und mit dem Lenkrad verbunden ist, solange eine Positionierung der Fingersensoreinrichtung im Bereich zwischen Lenkradspeiche und Lenkradkörper in bevorzugter Weise auf der Rückseite des Lenkrads erfolgt. Gemäß der Erfindung ist die Fingersensoreinrichtung in einem gegenüber dem Lenkradkörper federnd nachgiebig aufgehängten Sensorflächenbereich angeordnet.

Die Anordnung der Fingersensoreinrichtung in einem federnd nachgiebigen Sensorflächenbereich des Lenkrads ist insofern besonders vorteilhaft, als auf diese Weise eine zuverlässige, reproduzierbare Anlage des bzw. der Finger des Fahrers an der Sensorfläche gewährleistet werden kann, da die federbelastete Nachgiebigkeit des Sensorflächenbereichs dem Fahrer ein haptisches Feedback darüber vermittelt, ob bereits ein genügender Anpressdruck des bzw. der Finger an der Sensorfläche vorliegt oder nicht.

Die Erfindung kann ferner auch unabhängig davon verwirklicht werden, wie die federnde Nachgiebigkeit des Sensorflächenbereichs am Lenkrad konstruktiv realisiert wird. So ist beispielsweise eine einstückig elastisch an der Lenkradverkleidung angeordnete Ausbildung der Sensorfläche denkbar. Für eine deutlich spürbare und reproduzierbare haptische Rückmeldung bezüglich des korrekten Anpressdrucks an der Sensorfläche ist gemäß der Erfindung vorgesehen, dass der Sensorflächenbereich durch die Betätigungsfläche eines Tastschalters gebildet ist. Auf diese Weise kann sowohl die Betätigungskraft als auch der Betätigungsweg der federnd nachgiebig am Lenkrad angeordneten Sensorfläche konstruktiv exakt festgelegt werden, und sowohl Kraft als auch Weg bleiben zudem dauerhaft reproduzierbar erhalten. Vorzugsweise ist der Tastschalter dabei zudem so eingerichtet, dass bei entsprechender Betätigung, also beim Niederdrücken des Tastschalters, der Messvorgang automatisch ausgelöst wird. Auch hierdurch wird sichergestellt, dass die Messung erst bei ausreichender Anpresskraft des bzw. der Finger im Bereich der Messsensoren durchgeführt wird.

Gemäß einer weiteren bevorzugten Ausführungsform der Erfindung ist vorgesehen, dass der Sensorflächenbereich zumindest ein vertieftes Fingerbett zur Aufnahme zumindest einer Daumen - oder Fingerkuppe umfasst. Dabei ist in dem vertieften Fingerbett die optische Sensorvorrichtung bündig angeordnet. Das im Sensorflächenbereich angeordnete vertiefte Fingerbett mit der darin angeordneten Sensoreinrichtung verbessert zusätzlich die Haptik bezüglich der korrekten Anlage des Fingers im Bereich der Sensoreinrichtung, und erhöht somit weiter die Zuverlässigkeit der Messung der Vitalparameter.

Eine weitere besonders bevorzugte Ausführungsform der Erfindung sieht vor, dass der Sensorflächenbereich im Wesentlichen flächig paddelförmig ausgebildet ist. Dies erleichtert die gleichzeitige Anlage mehrerer Finger einer Hand, wodurch der Komfort beim Halten des Lenkrads verbessert wird insbesondere dann, wenn die Finger der Hand über längere Zeit im Bereich der Sensorflächen ruhen. Vorzugsweise ist dabei der Sensorflächenbereich zudem mit einer elektrisch leitfähigen Oberfläche versehen, wobei die elektrisch leitfähige Oberfläche mit einer Auswertungseinrichtung zur Erstellung eines Elektrokardiogramms verbunden ist.

Dank dieser Ausführungsform kann neben der Messung bzw. Überwachung von Puls- bzw. Blutsauerstoffwerten zusätzlich auch ein Elektrokardiogramm des Fahrers erstellt werden, wodurch sich eine noch zuverlässigere Erfassung bzw. ein entsprechendes Monitoring der Vitalparameter, und damit des Gesundheitszustands des Fahrers erzielen lässt.

Eine weitere besonders bevorzugte Ausführungsform der Erfindung sieht vor, dass die Erfassungsvorrichtung zwei am Lenkrad angeordnete Fingersensoreinrichtungen aufweist, die jeweils einer Hand des Fahrers zugeordnet und dementsprechend symmetrisch am Lenkrad angeordnet sind. Auf diese Weise kann die Zuverlässigkeit und Genauigkeit der Messung der Vitalparameter - beispielsweise durch Mittelwertbildung - weiter verbessert werden. Das Vorhandensein von zwei Sensoreinrichtungen kommt zudem insbesondere der EKG-Messung zugute.

Eine weitere bevorzugte Ausführungsform der Erfindung sieht vor, dass die optische Sensorvorrichtung neben dem lichtempfindlichen Element eine erste Lichtquelle zur Erzeugung von Licht im sichtbaren Wellenlängenbereich, sowie eine zweite Lichtquelle zur Erzeugung von Licht in einem nicht sichtbaren Wellenlängenbereich, insbesondere im Infrarot-Wellenlängenbereich umfasst. Auf diese Weise können mit der erfindungsgemäßen Vitalparameter-Erfassungsvorrichtung sowohl gefäßvolumenbezogene Messungen, insbesondere in Form der optischen Plethysmographie, als auch Messungen im Hinblick auf die Blutzusammensetzung, - insbesondere in Form der Sauerstoffmessung mittels optischer Pulsoxymetrie - durchgeführt werden.

Eine weitere Ausführungsform der Erfindung sieht vor, dass der Tastschalter als Betätigungseinrichtung für eine weitere Funktion des Kraftfahrzeugs eingerichtet ist. Vorzugsweise bildet der Tastschalter dabei ein Schaltpaddel für ein Gangwechselgetriebe.

Die Unterscheidung, ob eine Betätigung bzw. ein Niederdrücken des Tastschalters als Auslöser zur Erfassung der Vitalparameter, oder zur Betätigung der weiteren Funktion, insbesondere zur Auslösung eines Gangwechsels gewertet werden soll, kann dabei auf unterschiedliche Weise erfolgen. So kann beispielsweise das Niederdrücken nur eines von zwei vorhandenen Tastschaltern bzw. Schaltpaddeln grundsätzlich als Schaltbefehl gewertet werden, während das Niederdrücken beider vorhandener Tastschalter bzw. Schaltpaddel grundsätzlich als Befehl zur Auslösung der Vitalparameter-Erfassung gewertet werden kann. Alternativ kann am Lenkrad beispielsweise eine Zusatztaste vorgesehen sein, bei deren Niederdrücken die anschließende oder gleichzeitige Betätigung des bzw. der Schaltpaddel nicht als Schaltbefehl, sondern als Befehl zur Vitalparameter-Erfassung gewertet wird.

Mit der vorliegenden Erfindung kann eine Erfassungsvorrichtung bereitgestellt werden, die eine zuverlässige und umfassende Messung und Überwachung der Vitalparameter auch während der Fahrt erlaubt, ohne Fahrkomfort bzw. Fahrsicherheit zu beeinträchtigen, insbesondere ohne dass eine Hand vom Lenkrad genommen werden muss. Durch die bevorzugte Ausgestaltung und Anordnung der Fingersensoreinrichtung kann ferner eine hohe Messgenauigkeit und geringe Beeinträchtigung durch schwankende Umgebungsbedingungen wie Licht und Temperatur erreicht werden.

Weitere Merkmale und Vorteile der vorliegenden Erfindung ergeben sich aus der nachfolgenden Beschreibung von bevorzugten Ausführungsbeispielen der Erfindung, anhand der Figuren der Zeichnung, die bevorzugte erfindungsrelevante Einzelheiten zeigen, und aus den Patentansprüchen.

Bevorzugte Ausführungsbeispiele der vorliegenden Erfindung werden nachfolgend anhand der beigefügten Zeichnung näher erläutert. Es zeigen:
- Fig. 1: in einer Frontalansicht ein Lenkrad eines Kraftfahrzeugs mit einer Erfassungsvorrichtung zur Erfassung von Vitalparametern gemäß einem bevorzugten Ausführungsbeispiel der vorliegenden Erfindung;
- Fig. 2: das in Fig. 1 gezeigte Lenkrad mit der Erfassungsvorrichtung in einer Rückansicht;
- Fig. 3: in einer Ausschnittsdarstellung das Lenkrad mit der Erfassungsvorrichtung gemäß Fig. 1 und 2 in der Draufsicht; und
- Fig. 4: in einer vergrößerten, isometrischen Darstellung ein Sensorpaddel der Erfassungsvorrichtung gemäß Fig. 1 und 2.

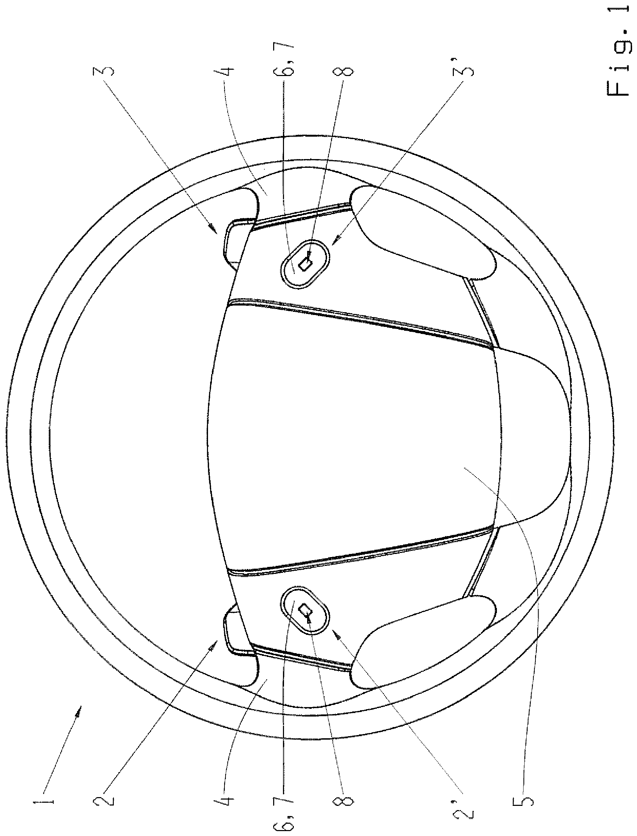

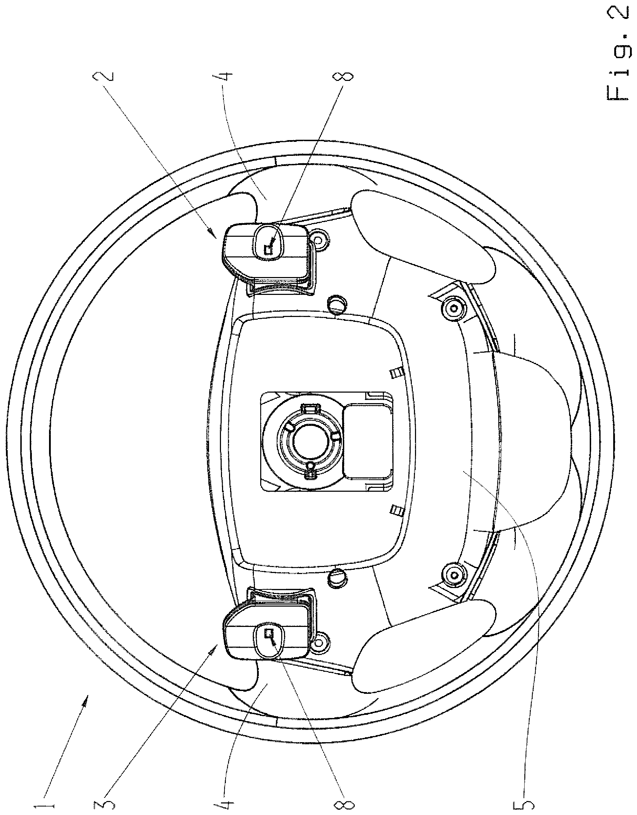

Die Fig. 1 und 2 zeigen ein Lenkrad 1 eines Kraftfahrzeugs aufweisend eine Erfassungsvorrichtung gemäß einem bevorzugten Ausführungsbeispiel der vorliegenden Erfindung. In einer Zusammenschau der Fig. 1 und 2 wird dabei zunächst einmal ersichtlich, dass das Lenkrad 1 bei der dargestellten Ausführungsform zwei Fingersensoreinrichtungen 2 und 3 umfasst. Dabei sind die Fingersensoreinrichtungen 2 und 3 auf der dem Fahrer abgewandten Rückseite des Lenkrads 1, jeweils im Übergangsbereich zwischen einer Lenkradspeiche 4 und der Rückseite des Lenkradkörpers bzw. Lenkrad-Pralltopfs 5 angeordnet.

Man erkennt, dass die Positionierung der Sensoreinrichtungen 2, 3 es erlaubt, die erforderlichen Messungen der Vitalparameter des Fahrers mit geringstmöglichem Aufwand beispielsweise während der Fahrt durchzuführen, ohne dass eine Hand vom Lenkrad 1 genommen werden muss. Die Messungen können dabei typischerweise erfolgen, ohne dass die Haltung der Hände am Lenkrad 1 verändert werden muss, da das Lenkrad 1 erfahrungsgemäß zumeist im Bereich der beiden horizontalen Speichen 4 gehalten wird. Zur Messung der Vitalparameter müssten somit lediglich die Finger auf die hier als Sensorpaddel 2, 3 ausgeführten Sensoreinrichtungen gelegt werden (falls sich die Finger nicht bereits dort befinden sollten), und die Messung kann ausgelöst werden.

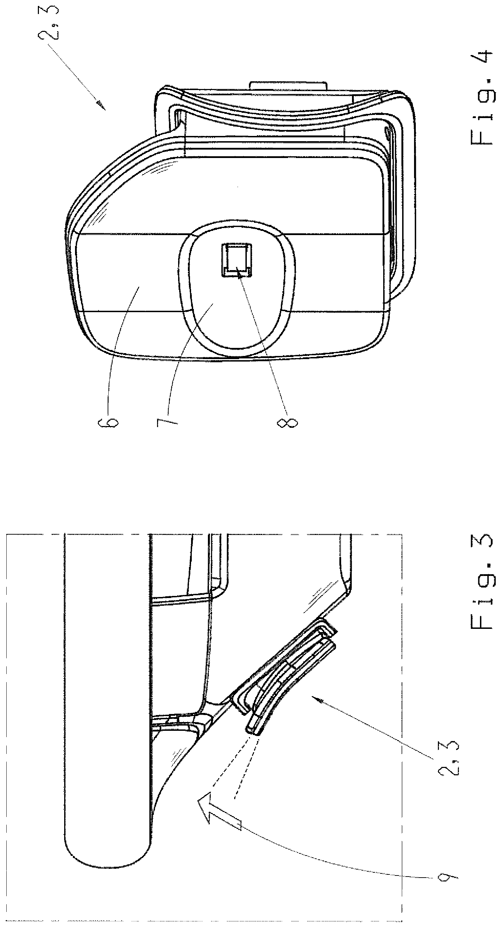

Fig. 3 zeigt eine der beiden Sensoreinrichtungen 2, 3 des Lenkrads 1 gemäß Fig. 1 und 2 nochmals im Einbauzustand in einer vergrößerten Draufsicht, während Fig. 4 die Sensoreinrichtung 2, 3 nochmals separat in isometrischer Darstellung zeigt. Hierbei wird erkennbar, dass die Sensoreinrichtung 2, 3 als Modul in Form eines Tastschalters bzw. Schaltpaddels 2, 3 ausgebildet ist, wodurch somit die Betätigungsoberfläche 6 des Tastschalters 2, 3 gleichzeitig die Kontaktfläche für die Finger bildet, und insbesondere das vertiefte Fingerbett 7 zur Aufnahme einer Fingerkuppe zum Zweck der optischen Vitalparameter-Erfassung enthält. In dem Fingerbett 7 ist hierzu eine optische Sensoreinrichtung 8 angeordnet, welche die zur Messung erforderlichen Leuchtdioden bzw. Fotosensoren enthält.

Die Vitalparameter-Erfassung bzw. -Messung erfolgt bei dem dargestellten Ausführungsbeispiel der Erfindung dadurch, dass vorzugsweise drei Finger einer Hand auf die Betätigungsoberfläche 6 des bzw. der Tastschalter 2, 3 gelegt werden, wobei der mittlere der drei Finger in dem vertieften Fingerbett 7 zu liegen kommt und damit in unmittelbaren Kontakt mit der optischen Sensoreinrichtung 8 steht. Die eigentliche Messung wird sodann durch leichten Druck auf die Betätigungsoberfläche 6 des Tastschalters 2, 3 ausgelöst, wodurch der Tastschalter 2, 3 wie durch den Pfeil 9 symbolisiert niedergedrückt wird, was wiederum durch Betätigung eines elektrischen Kontakts innerhalb des Tastschalters 2, 3 die Messung auslöst.

Die Integration der Sensoreinrichtung 8 in einen Tastschalter 2, 3 ist somit in zweifacher Hinsicht vorteilhaft. Einerseits erfolgt die Messung kontrolliert und zu einem spezifischen Zeitpunkt nur dann, wenn der Tastschalter 2, 3 niedergedrückt wird. Andererseits führt die Notwendigkeit des Niederdrückens des Tastschalters 2, 3 - um auf diese Weise die Messung auszulösen - ihrerseits dazu, dass ein definierter, inniger Kontakt zwischen der Hautoberfläche und dem Messsensor 8 immer dann tatsächlich besteht, wenn die Messung durchgeführt werden soll.

Zudem kann bei dem dargestellten Ausführungsbeispiel - gleichzeitig mit der optischen Messung von blutkreislaufbezogenen Parametern mittels der optischen Sensoreinrichtung 8 - auch noch eine EKG-Messung durchgeführt werden. Hierzu sind die Oberflächen 6 der Tastschalter 2, 3 aus Metall gebildet bzw. mit einer leitfähigen Oberflächenschicht versehen. Auf diese Weise kann mittels elektronischer Auswertung der zwischen den Fingeroberflächen und den Oberflächen 6 der Tastschalter 2, 3 fließenden Kontaktströme ein entsprechendes Elektrokardiogramm erstellt und in das Monitoring des Gesundheitszustands des Fahrers einbezogen werden.

In Fig. 1 zusätzlich (strichliert) eingezeichnet ist eine alternative Anordnung der Sensoreinrichtungen 2', 3' auf der dem Fahrer zugewandten Vorderseite des Lenkrads 1 gezeigt. Die Sensoreinrichtungen 2', 3' umfassen auch hier wieder eine leitfähige Betätigungsoberfläche 6 mit einem darin angeordneten Fingerbett 7, wobei im Fingerbett 7 wiederum die eigentliche optische Sensoreinrichtung 8 angeordnet ist. Die Sensoreinrichtungen 2', 3' bzw. die zugehörigen Fingerbetten 7 sind für den Fall der Anordnung der Sensoreinrichtungen 2', 3' auf der dem Fahrer zugewandten Seite des Lenkrads 1 so ausgeformt bzw. ausgerichtet, dass die Daumen jeweils in die Fingerbetten 7 eingelegt werden können, ohne dass hierzu die Standardposition der Hände am Lenkrad 1 verändert werden muss.

Eine weitere (nicht dargestellte) Möglichkeit für die Anordnung der Sensoreinrichtungen 2, 3 bzw. 2', 3', die nicht Teil der vorliegenden Erfindung bildet, besteht darin, die Sensoreinrichtungen mit deren leitfähigen Betätigungsflächen 6, Fingerbetten 7 und optischen Sensoreinrichtungen 8 am Lenkradkranz so anzuordnen, dass die Sensoreinrichtungen jeweils wieder mit einem oder mehreren Fingern erreichbar sind, ohne dass hierzu die Hände vom Lenkrad 1 genommen werden müssen, bzw. ohne dass die Standardposition der Hände am Lenkrad 1 verändert werden muss.

Im Ergebnis wird somit deutlich, dass mit der Erfindung eine Erfassungsvorrichtung zur Erfassung von Vitalparametern geschaffen wird, die eine besonders zuverlässige Messung und Überwachung der Vitalparameter insbesondere auch während der Fahrt in einem Automobil ermöglicht, wobei gleichzeitig - auch während der Durchführung der Messungen - eine Beeinträchtigung von Fahrkomfort oder Fahrsicherheit reduziert bzw. eliminiert werden kann.

### Bezugszeichen

- 1: Lenkrad
- 2, 3: Fingersensoreinrichtung, Tastschalter, Schaltpaddel
- 2', 3': Fingersensoreinrichtung, Tastschalter
- 4: Lenkradspeiche
- 5: Pralltopf, Lenkradkörper
- 6: Sensorflächenbereich, Betätigungsoberfläche, EKG-Kontakt
- 7: Fingerbett
- 8: optische Sensoreinrichtung
- 9: Taster-Betätigungsweg

## Patentansprüche

1. Erfassungsvorrichtung zur Erfassung zumindest eines Vitalparameters einer Person in einem Kraftfahrzeug mit einem Lenkrad (1), die Erfassungsvorrichtung, umfassend zumindest eine Fingersensoreinrichtung mit zumindest einer optischen Sensorvorrichtung (8), wobei die optische Sensorvorrichtung (8) zumindest eine erste Lichtquelle sowie zumindest ein lichtempfindliches Element umfasst, wobei die Fingersensoreinrichtung am Lenkrad (1) im Übergangsbereich zwischen einer Lenkradspeiche (4) und dem Lenkradkörper (5) anordnbar ist, und wobei die Fingersensoreinrichtung in einem gegenüber dem Lenkradkörper (5) federnd nachgiebig aufhängbaren Sensorflächenbereich (6) angeordnet ist; **dadurch gekennzeichnet, dass** die Erfassungsvorrichtung außerdem einen der Fingersensoreinrichtung zugeordneten Tastschalter (2, 3) umfasst und dass der Sensorflächenbereich (6) durch die Betätigungsfläche (6) des Tastschalters (2, 3) gebildet ist.

2. Erfassungsvorrichtung nach Anspruch 1, **dadurch gekennzeichnet, dass** die Fingersensoreinrichtung auf der dem Fahrer abgewandten Rückseite des Lenkrads (1) anordnbar und zur Kontaktierung zumindest eines Fingers eingerichtet ist.

3. Erfassungsvorrichtung nach Anspruch 1, **dadurch gekennzeichnet, dass** die Fingersensoreinrichtung auf der dem Fahrer zugewandten Vorderseite des Lenkrads (1) anordnbar und zur Kontaktierung eines Daumens eingerichtet ist.

4. Erfassungsvorrichtung nach einem der vorherigen Ansprüche, **dadurch gekennzeichnet, dass** der Tastschalter (2, 3) zur Auslösung des Messvorgangs durch die Erfassungsvorrichtung eingerichtet ist.

5. Erfassungsvorrichtung nach einem der vorherigen Ansprüche, **dadurch gekennzeichnet, dass** der Sensorflächenbereich (6) zumindest ein vertieftes Fingerbett (7) zur Aufnahme zumindest einer Daumen- oder Fingerkuppe umfasst, wobei in dem Fingerbett (7) die optische Sensorvorrichtung (8) bündig angeordnet ist.

6. Erfassungsvorrichtung nach einem der vorherigen Ansprüche, **dadurch gekennzeichnet, dass** der Sensorflächenbereich (6) im Wesentlichen flächig paddelförmig ausgebildet ist.

7. Erfassungsvorrichtung nach einem der vorherigen Ansprüche, **dadurch gekennzeichnet, dass** der Sensorflächenbereich (6) eine elektrisch leitfähige Oberfläche aufweist, wobei die elektrisch leitfähige Oberfläche mit einer Auswertungseinrichtung verbunden ist, die zur Erstellung eines Elektrokardiogramms eingerichtet ist.

8. Erfassungsvorrichtung nach einem der vorherigen Ansprüche, **dadurch gekennzeichnet, dass** die Erfassungsvorrichtung zwei symmetrisch am Lenkrad anordnbare Fingersensoreinrichtungen und zugeordnete Tastschalter (2, 3) aufweist, wobei jede der beiden Fingersensoreinrichtungen (2, 3) jeweils einer Hand des Fahrers zugeordnet ist.

9. Erfassungsvorrichtung nach einem der vorherigen Ansprüche, **dadurch gekennzeichnet, dass** die optische Sensorvorrichtung (8) eine erste Lichtquelle zur Erzeugung von Licht im sichtbaren Wellenlängenbereich sowie eine zweite Lichtquelle zur Erzeugung von Licht in einem nicht-sichtbaren Wellenlängenbereich sowie das zumindest eine lichtempfindliche Element umfasst.

10. Erfassungsvorrichtung nach einem der vorherigen Ansprüche, **dadurch gekennzeichnet, dass** der Tastschalter (2, 3) als Betätigungseinrichtung für eine weitere Funktion des Kraftfahrzeugs eingerichtet ist.

11. Erfassungsvorrichtung nach Anspruch 10, **dadurch gekennzeichnet, dass** der Tastschalter (2, 3) ein Schaltpaddel für ein Gangwechselgetriebe ist.

## Claims

1. Detection device for detecting at least one vital parameter of a person in a motor vehicle having a steering wheel (1), the detection device comprising at least one finger sensor having at least one optical sensor device (8), the optical sensor device (8) comprising at least one first light source and at least one light-sensitive element, wherein the finger sensor can be arranged on the steering wheel (1) in the transition area between a steering wheel spoke (4) and the steering wheel body (5), and the finger sensor being arranged in a sensor surface area (6) that can be suspended in a sprung compliant manner with respect to the steering wheel body (5), **characterized in that** the detection device additionally comprises a pushbutton switch (2, 3) assigned to the finger sensor, and **in that** the sensor surface area (6) is formed by the actuating surface (6) of the pushbutton switch (2, 3).

2. Detection device according to Claim 1, **characterized in that** the finger sensor can be arranged on the rear side of the steering wheel (1), facing away from the driver, and is configured so as to make contact with at least one finger.

3. Detection device according to Claim 1, **characterized in that** the finger sensor can be arranged on the front side of the steering wheel (1), facing the driver, and is configured so as to make contact with a thumb.

4. Detection device according to one of the preceding claims, **characterized in that** the pushbutton switch (2, 3) is configured so as to trigger the measuring operation by the detection device.

5. Detection device according to one of the preceding claims, **characterized in that** the sensor surface area (6) comprises at least one depressed finger cup (7) to receive at least one tip of a thumb or of a finger, the optical sensor device (8) being arranged flush in the finger cup (7).

6. Detection device according to one of the preceding claims, **characterized in that** the sensor surface area (6) is designed to be shaped substantially like a flat paddle.

7. Detection device according to one of the preceding claims, **characterized in that** the sensor surface area (6) has an electrically conductive surface, the electrically conductive surface being connected to an evaluation means which is configured to create an electrocardiogram.

8. Detection device according to one of the preceding claims, **characterized in that** the detection device has two finger sensors and associated pushbutton switches (2, 3) that can be arranged symmetrically on the steering wheel, each of the two finger sensors (2, 3) being assigned respectively to a hand of the driver.

9. Detection device according to one of the preceding claims, **characterized in that** the optical sensor device (8) comprises a first light source for producing light in the visible wavelength range and a second light source for producing light in a non-visible wavelength range, and the at least one light-sensitive element.

10. Detection device according to one of the preceding claims, **characterized in that** the pushbutton switch (2, 3) is configured as an actuating device for a further function of the motor vehicle.

11. Detection device according to Claim 10, **characterized in that** the pushbutton switch (2, 3) is a switch paddle for a gear-change transmission.

## Revendications

1. Dispositif de détection destiné à détecter au moins un paramètre vital d'une personne dans un véhicule automobile comportant un volant de direction (1), le dispositif de détection comprenant au moins un moyen capteur de doigt comportant au moins un dispositif capteur optique (8), dans lequel le dispositif capteur optique (8) comprend au moins une première source de lumière ainsi qu'au moins un élément sensible à la lumière, dans lequel le moyen capteur de doigt peut être disposé sur le volant de direction (1) dans une zone de transition entre un bras de volant de direction (4) et le corps de volant (5), et dans lequel le moyen capteur de doigt est disposé dans une zone de surface de capteur (6) pouvant être suspendue de manière souple et élastique par rapport au corps de volant de direction (5) ; **caractérisé en ce que** le dispositif de détection comprend en outre un commutateur à touche (2, 3) associé au moyen capteur de doigt et **en ce que** la zone de surface de capteur (6) est formée par la surface d'actionnement (6) du commutateur à touche (2, 3).

2. Dispositif de détection selon la revendication 1, **caractérisé en ce que** le moyen capteur de doigt peut être disposé sur la face arrière du volant de direction (1) qui est tournée en sens opposé au conducteur et **en ce qu'**il est conçu pour être en contact avec au moins un doigt.

3. Dispositif de détection selon la revendication 1, **caractérisé en ce que** le moyen capteur de doigt peut être disposé sur la face avant du volant de direction (1) qui est tournée vers le conducteur et **en ce qu'**il est conçu pour être mis en contact avec un pouce.

4. Dispositif de détection selon l'une quelconque des revendications précédentes, **caractérisé en ce que** le commutateur à touche (2, 3) est conçu pour déclencher le processus de mesure par l'intermédiaire du dispositif de détection.

5. Dispositif de détection selon l'une quelconque des revendications précédentes, **caractérisé en ce que** la zone de surface de capteur (6) comprend au moins un réceptacle de doigt en creux (7) destiné à recevoir au moins une pulpe d'un pouce ou d'un doigt, dans lequel le dispositif capteur optique (8) est disposé de manière à affleurer dans le réceptacle de doigt (7).

6. Dispositif de détection selon l'une quelconque des revendications précédentes, **caractérisé en ce que** la zone de surface de capteur (6) est réalisée de manière à présenter sensiblement la forme d'une palette plane.

7. Dispositif de détection selon l'une quelconque des revendications précédentes, **caractérisé en ce que** la zone de surface de capteur (6) présente une surface électriquement conductrice, dans lequel la surface électriquement conductrice est reliée à un moyen d'évaluation qui est conçu pour établir un électrocardiogramme.

8. Dispositif de détection selon l'une quelconque des revendications précédentes, **caractérisé en ce que** le dispositif de détection comporte deux moyens capteurs de doigt pouvant être disposés symétriquement sur le volant de direction et des commutateurs à touches (2, 3) associés, dans lequel chacun des moyens capteurs de doigts (2, 3) est associé à une main du conducteur.

9. Dispositif de détection selon l'une quelconque des revendications précédentes, **caractérisé en ce que** le dispositif capteur optique (8) comprend une source de lumière destinée à générer de la lumière dans une plage de longueurs d'onde visibles, une deuxième source de lumière destinée à générer une lumière se situant dans une plage de longueurs d'onde non visible, ainsi qu'au moins un élément sensible à la lumière.

10. Dispositif de détection selon l'une quelconque des revendications précédentes, **caractérisé en ce que** le commutateur à touche (2, 3) est conçu en tant que dispositif d'actionnement pour une fonction supplémentaire du véhicule automobile.

11. Dispositif de détection selon la revendication 10, **caractérisé en ce que** le commutateur à touche (2, 3) est une palette de commande destinée à une boîte de vitesses.
